# FASCICULE DE BREVET EUROPEEN

(11) **EP 2 341 975 B1**
(45) Date de publication et mention de la délivrance du brevet: **05.06.2019**
(21) Numéro de dépôt: 09784272.8
(22) Date de dépôt: 16.07.2009
(51) Int. Cl.: F16L 25/10, A61M 39/10

(54) **RACCORD FLUIDIQUE MALE POLYVALENT POUR DISPOSITIF DE RACCORDEMENT, ET UN TEL DISPOSITIF L'INCORPORANT**
FLUIDISCHER UNIVERSALSTECKER FÜR EINE KOPPLUNGSVORRICHTUNG UND DERARTIGE VORRICHTUNG DAMIT
MULTIPURPOSE MALE FLUIDIC COUPLING FOR A COUPLING DEVICE AND DEVICE SUCH AS THIS INCORPORATING IT

(30) Priorité: 18.07.2008 FR 0804125
(43) Date de publication de la demande: 13.07.2011
(73) Titulaire: Commissariat à l'Énergie Atomique et aux Énergies Alternatives, 75015 Paris (FR)
(72) Inventeur: REYMOND, Jean-Marc, F-78470 Saint-Remy-les-Chevreuse (FR); HAMELIN, Annabelle, F-91530 Saint-Cheron (FR)
(74) Mandataire: Gevers & Orès
(86) Numéro de dépôt international: PCT/FR2009/000871
(87) Numéro de publication internationale: WO 2010/007254

(56) Documents cités:
- EP-A- 0 978 292
- EP-A- 2 005 882
- DE-A1- 4 403 630
- FR-A- 2 395 037
- US-A- 5 964 737

## Description

La présente invention concerne un raccord fluidique mâle à emmancher dans un raccord femelle raccordé à un premier conduit avec lequel est destiné à communiquer un second conduit traversant le raccord mâle, pour former un dispositif de raccordement fluidique exempt de volume mort apte à transférer un fluide, et un tel dispositif. L'invention s'applique plus particulièrement, mais non exclusivement, au transfert de microéchantillons liquides à prélever ou à injecter.

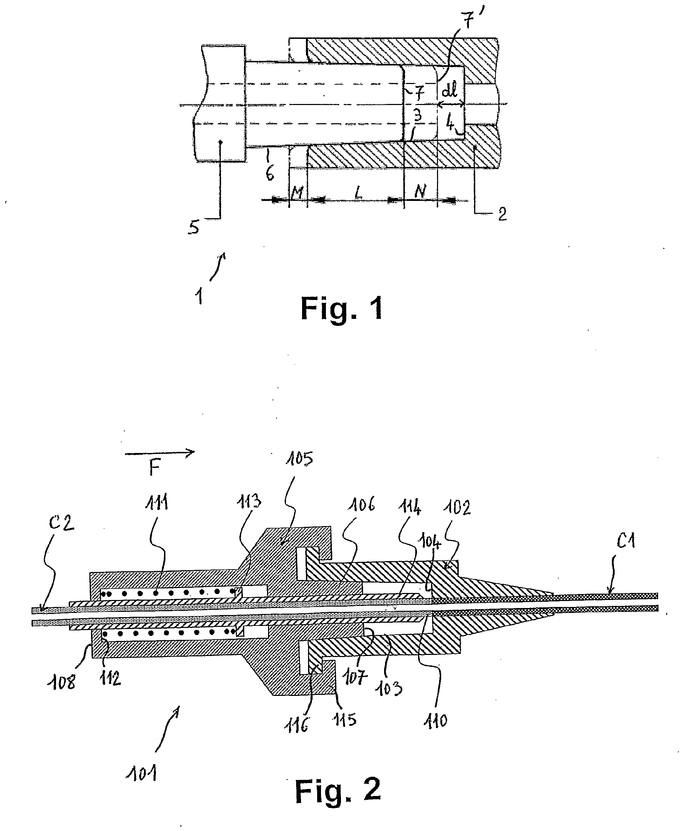

Les dispositifs de raccordement fluidiques qui sont utilisés en médecine et en biologie en relation avec un cathéter ou similaire, d'une part, et avec un micro-tube souple, d'autre part, sont usuellement des raccords standardisés de type « Luer » définis par la norme ISO 59461 de 1986, ou bien de type « Luer Lock » définis par la norme ISO 594-2 de 1998. En référence à la figure 1 annexée à la présente description, les dispositifs de raccordement usuels 1 comportent essentiellement :
- un raccord femelle 2 dans lequel est destiné à être enfoncé un cathéter et qui comprend une surface interne d'emmanchement conique 3 convergeant vers une extrémité femelle 4 où débouche le cathéter, et
- un raccord mâle 5 qui est emmanché dans le raccord femelle 2 via sa surface externe conique 6 avec la même conicité que la surface interne 3 du raccord femelle, et qui converge vers une extrémité mâle 7 en laissant à l'état d'enfoncement maximal une distance axiale dl avec l'extrémité femelle 4. A titre d'exemple, on a représenté sur la figure 1 la position extrême d'enfoncement 7' que peut avoir l'extrémité mâle 7 ; elle laisse alors un espace libre (volume mort) dont la profondeur axiale est dl, minimale lorsque la surface 7 occupe la position 7'.

Le tableau 1 ci-après recense les principales caractéristiques dimensionnelles des dispositifs de raccordement de type « Luer » (angle de conicité de 6 %) en fonction du matériau utilisé, selon la norme précitée.

**Tableau 1 :**

| Cote | | Désignation | Dimensions (mm) | |
|---|---|---|---|---|
| | | | Matériau rigide | Matériau semi-rigide |
| Dimensions de base | dₘᵢₙ | Diamètre minimal de l'extrémité du raccord conique mâle (diamètre de référence) | 3,925 | 3,925 |
| | dₘₐₓ | Diamètre maximal de l'extrémité du raccord conique mâle | 3,990 | 4,027 |
| | Dₘᵢₙ | Diamètre minimal de l'ouverture du raccord conique femelle | 4,270 | 4,270 |
| | Dₘₐₓ | Diamètre maximal de l'ouverture du raccord conique femelle | 4,315 | 4,315 |
| | E | Longueur minimale du raccord conique mâle | 7,500 | 7,500 |
| | F | Profondeur minimale du raccord conique femelle | 7,500 | 7,500 |
| Autres dimensions | L* | Pénétration minimale | 4,665 | 4,050 |
| | M* | Ecart sur la pénétration du raccord femelle | 0,750 | 0,750 |
| | N* | Ecart sur la pénétration du raccord mâle | 1,083 | 1,700 |
| | R** ₘₐₓ | Rayon de courbure | 0,5 | 0,5 |

| | | | | |
|---|---|---|---|---|
| En référence aux symboles * et ** de ce tableau : * les dimensions L, M, N résultent des dimensions de base, et ** ou chanfrein d'entrée équivalent n'ayant pas d'angles vifs | | | | |

Un inconvénient majeur des dispositifs de raccordement définis par les normes précitées est que la distance dl (typiquement de 3 à 6 mm environ selon le type de raccord femelle utilisé) entre les extrémités respectives des raccords mâle et femelle génère une cavité formant un volume mort intrinsèque qui devient pénalisant dans diverses circonstances. La plus fréquente se présente lorsque les raccords servent à la circulation de très petits échantillons liquides, d'un ordre de grandeur comparable ou même inférieur à ce volume mort. A titre d'exemple, lors de l'injection ou du prélèvement de microéchantillons de sang de mammifère, on utilise généralement un conduit souple muni d'un raccord d'un diamètre d'environ 4 mm, l'espace ainsi délimité déterminant un volume mort de l'ordre de 35 µL (pour une cavité de 3 mm de longueur) à 70 µL (pour une cavité de 6 mm de longueur), lequel volume mort peut engendrer les problèmes suivants en utilisation du fait de ces dimensions relativement élevées :
- plusieurs microéchantillons successifs servant à remplir ce volume mort, cela retarde la traversée des premiers microéchantillons et implique de prélever un volume de fluide plus grand qui est perdu, et
- la section du conduit fluidique s'élargissant considérablement du fait de ce volume mort, différents microéchantillons s'y mélangent les uns aux autres, ce qui est pénalisant si le fluide est un liquide, et si l'on exploite ultérieurement ces microéchantillons par exemple à des fins d'analyse. Cela nuit à leur traçabilité et est particulièrement préjudiciable dans le cas du suivi de processus évolutifs dans le temps, comme par exemple des phénomènes biologiques rapides, au cours desquels il est fondamental que chaque microéchantillon puisse conserver ses caractéristiques initiales tout au long du processus.

Par ailleurs et comme le montre le tableau 1 ci-dessus, les normes relatives aux raccords « Luer » ne définissent pas un type unique de raccord, notamment de raccords femelles, mais une pluralité de types compatibles entre eux, et ayant des dimensions en partie définies dans ce tableau (ces normes ne précisent pas, par exemple, la dimension de la cavité entre les raccords mâle et femelle). Il en résulte que tout dispositif de raccordement de type « Luer » ou « Luer Lock » minimisant ce volume mort doit rechercher la compatibilité avec toutes les variantes dimensionnelles de ces divers types, afin de ne pas complexifier pour les utilisateurs la gestion des approvisionnements en raccords.

Il est par ailleurs connu par le document US-A-4 966 588 d'utiliser, pour l'injection d'une substance liquide thérapeutique, un dispositif de raccordement fluidique comportant essentiellement :
- un raccord mâle emmanché dans un raccord femelle via des surfaces respectives d'emmanchement cylindriques munies d'épaulements, ce raccord femelle étant destiné à recevoir une canule formant embout d'injection, et
- une aiguille rigide d'injection qui est insérée de manière traversante à la fois dans le raccord mâle, le raccord femelle et la canule en venant percer une rondelle d'étanchéité positionnée entre les deux raccords, et qui est destinée à être implantée dans le corps à traiter.

Ce dispositif de raccordement à aiguille d'injection rigide traversant de part en part les raccords et la canule permet bien de minimiser le volume mort, mais il présente les inconvénients suivants :
- le diamètre de l'aiguille est faible en comparaison de celui de l'ouverture du raccord mâle et de la conduite fluidique, ce qui réduit significativement le débit et se traduit par une augmentation de la vitesse du fluide (la viscosité de ce dernier peut alors poser problème) ;
- ce dispositif peut n'être efficace que pour un seul raccordement, car rien ne permet de penser que des raccordements ultérieurs feraient passer l'aiguille dans le même orifice de la rondelle d'étanchéité (cette dernière pouvant être déplacée involontairement suite au retrait de l'aiguille après chaque injection), ni que cette rondelle présenterait alors la même étanchéité ;
- ce dispositif n'est pas conforme aux types précités « Luer » ou « Luer Lock » ; et
- l'aiguille dépasse nécessairement de la rondelle d'étanchéité d'une longueur pouvant gêner certaines applications en amont ou en aval de cette aiguille (par exemple lorsque la canule est constituée d'un cathéter souple, l'aiguille peut dépasser de l'extrémité du cathéter en lui faisant perdre sa souplesse et en risquant ainsi d'occasionner d'éventuelles lésions aux tissus biologiques environnants).

L'évolution actuelle des techniques biomédicales rend de plus en plus fréquent le recours à des microéchantillons liquides (i.e. des échantillons présentant chacun un volume inférieur à 100 µL et de préférence inférieur ou égal à 30 µL, comme par exemple des échantillons sanguins prélevés sur des petits animaux). Or, les lignes de prélèvement qui comportent au moins un dispositif de raccordement de type « Luer » ou « Luer Lock » doivent pouvoir conserver la traçabilité de ces microéchantillons, en permettant de retrouver en sortie les microéchantillons tels qu'ils étaient en entrée. Ces raccords doivent en outre présenter une étanchéité qui n'est pas significativement affectée par la force de serrage entre les raccords mâle et femelle, ni altérée par une succession de plusieurs montages et démontages.

Le document DE-A1-44 03 630 présente un assemblage complexe de plusieurs tuyaux et raccords mâle et femelle dans lequel c'est le raccord femelle (voir figure 3) qui est modifié pour minimiser le volume mort, étant précisé que le raccord mâle est ici vissé et donc non emmanché dans ce raccord femelle.

Le document US-A-5 964 737 présente à sa figure 19 une seringue se terminant par un embout mâle que l'on emmanche dans un embout femelle mais qui est notamment dépourvu de tout tube ou conduit souple le traversant.

Un but de la présente invention est de proposer un raccord fluidique mâle destiné à être emmanché dans un raccord fluidique femelle raccordé à un premier conduit avec lequel est destiné à communiquer un second conduit traversant le raccord mâle, pour former un dispositif de raccordement fluidique apte à transférer un fluide qui remédie à l'ensemble des inconvénients précités et qui permette également à un unique raccord mâle de s'adapter indifféremment à tous les types de raccords femelles définis par les normes précitées des raccords « Luer » et « Luer Lock », ce raccord femelle comprenant une surface radialement interne d'emmanchement se terminant par une extrémité radiale femelle où débouche ce premier conduit, le raccord mâle comprenant une surface radiale d'emmanchement se terminant par une première extrémité radiale mâle destinée à se trouver à l'intérieur du raccord femelle.

A cet effet, un dispositif selon l'invention est tel que ce second conduit est formé d'un tube souple qui est enfoncé dans le raccord mâle axialement au-delà de ladite première extrémité mâle et qui se termine par une extrémité libre apte à être plaquée de manière étanche contre ladite extrémité femelle par des moyens de rappel intégrés au raccord mâle, ces moyens de rappel coopérant lors de l'emmanchement avec des moyens de guidage et de maintien prévus dans le raccord mâle pour guider ce tube en coulissement axial à travers le raccord mâle en rapprochant ladite extrémité libre de ladite première extrémité mâle tout en maintenant cette extrémité libre contre ladite extrémité femelle, de sorte à supprimer tout volume mort entre le premier conduit et le raccord mâle.

On notera que ces moyens de rappel exercent, sur toute la course utile du tube, une force suffisante pour assurer l'étanchéité.

On notera également que les dispositifs de raccordement incorporant ce raccord mâle selon l'invention peuvent être parcourus par le fluide à transférer indifféremment dans un sens ou dans l'autre (i.e. pour une injection ou un prélèvement).

On notera en outre que si le fluide transféré est un liquide, les dispositifs de raccordement incorporant ce raccord mâle selon l'invention remédient aux inconvénients précités en relation avec le volume mort généré entre les raccords mâle et femelle, évitant ainsi notamment le mélange de microéchantillons.

Selon une autre caractéristique de l'invention, lesdits moyens de rappel peuvent être aptes à être comprimés axialement lors du coulissement du tube vers l'intérieur du raccord mâle, de telle sorte que ladite extrémité libre soit plaquée contre ladite extrémité femelle avec une force résultant au moins en partie de la compression des moyens de rappel due au débattement dudit tube lors de son coulissement, ce débattement étant prévu supérieur à la profondeur axiale du raccord femelle mesurée à partir de ladite extrémité femelle.

Afin que l'étanchéité soit effective dès le début de la course du tube à l'intérieur du raccord mâle, et que le débattement n'excède pas significativement la course utile pour les divers types de raccords femelles, ces moyens de rappel peuvent être avantageusement précontraints. En d'autres termes, ladite force de plaquage résulte alors non seulement de la compression desdits moyens de rappel lors du débattement du tube mais en outre d'une précontrainte appliquée à ces moyens de rappel avant montage du raccord mâle dans le raccord femelle, la précontrainte étant telle que, dès le début de son coulissement, ladite extrémité libre du tube est sensiblement apte à assurer l'étanchéité vis-à-vis du raccord femelle (i.e. est placée dans des conditions proches de celles assurant l'étanchéité), une compression réduite des moyens de rappel lors du coulissement permettant de conférer à la force de plaquage une intensité suffisante pour assurer l'étanchéité.

On notera que la précontrainte permet à cette force de varier peu, sur toute la course utile du tube à l'intérieur du raccord mâle. Or, plus la variation de cette force est faible, meilleure est la compatibilité du raccord mâle selon l'invention avec tous les types de raccords femelles notamment mentionnés dans les normes précitées.

Avec ou sans cette précontrainte, on conserve les spécifications fonctionnelles de ces normes et l'on rend l'étanchéité quasi-indépendante de la force de serrage entre les deux raccords et de l'usure résultant de plusieurs montages/démontages, du fait que l'extrémité libre du tube aboute sur l'extrémité radiale interne du raccord femelle avec une pression suffisante pour garantir l'étanchéité de cet aboutement grâce auxdits moyens de rappel. Cette pression résulte de la force créée par la compression des moyens de rappel, résultant du débattement du tube qui coulisse à l'intérieur du raccord et, éventuellement, de ladite précontrainte initiale.

Comme indiqué ci-dessus, on notera que cette polyvalence d'un raccord mâle selon l'invention avec divers raccords femelle de profondeurs axiales différentes est rendue possible par le dépassement du tube avant emmanchement d'une longueur suffisante par rapport à ladite première extrémité mâle, ce dépassement générant lors de l'emmanchement le retrait en sens inverse de ce tube qui se traduit par la poussée assurée par les moyens de rappel venant plaquer le tube contre ladite extrémité femelle.

Selon une autre caractéristique de l'invention, lesdits moyens de rappel peuvent être montés en butée, d'une part, contre une paroi radiale d'extrémité du raccord mâle opposée à ladite première extrémité mâle et, d'autre part, contre au moins une portée radiale qui est solidaire dudit tube. Avantageusement, lesdits moyens de rappel peuvent comprendre un ressort hélicoïdal à compression monté radialement entre ledit tube et ce raccord mâle.

Selon une autre caractéristique avantageuse de l'invention, lesdits moyens de guidage et de maintien peuvent être conçus pour rigidifier ledit tube sur une portion rigidifiée de ce dernier s'étendant axialement à l'intérieur du raccord mâle et se terminant au-delà de ladite première extrémité mâle à distance de ladite extrémité libre, de sorte que ce tube présente une portion souple d'extrémité de rigidité inférieure à celle de cette portion rigidifiée et apte à assurer l'étanchéité du raccordement entre ladite extrémité libre et ladite extrémité femelle en supportant la force de rappel générée par lesdits moyens de rappel contre ladite extrémité femelle.

On notera que cette portion rigidifiée, qui correspond à la partie du tube sollicitée par les moyens de rappel, permet d'éviter le flambage du tube à l'intérieur du raccord mâle, en relation avec cette portion souple d'extrémité qui présente des dimensions prévues pour éviter le flambage du tube dans la zone apicale de déformation élastique de ce dernier assurant l'étanchéité de ladite extrémité libre avec ladite extrémité femelle.

Selon un mode préférentiel de réalisation de l'invention, lesdits moyens de guidage et de maintien comprennent un manchon tubulaire rigide monté solidaire dudit tube en l'enserrant sur ladite portion rigidifiée, tel qu'un manchon métallique ou en matière plastique, la portion souple d'extrémité dudit tube étant dépourvue de ce manchon, lequel est monté coulissant axialement à l'intérieur du raccord mâle en traversant ladite première extrémité mâle et une seconde extrémité mâle opposée du raccord mâle. Ladite portée radiale pour lesdits moyens de rappel est dans ce cas formée par une collerette circonférentielle que présente ledit manchon.

Selon une variante de réalisation de l'invention, ladite portion rigidifiée peut faire partie intégrante dudit tube en présentant une rigidité supérieure à celle de ladite portion souple d'extrémité de ce tube, cette portion rigidifiée étant obtenue par un traitement de durcissement local du tube.

Selon une autre caractéristique de l'invention à la fois commune audit mode préférentiel et à cette variante, lesdits moyens de guidage et de maintien peuvent autoriser en outre une rotation libre dudit tube à l'intérieur du raccord mâle.

De préférence, ladite surface externe d'emmanchement du raccord mâle est une surface conique qui converge vers ladite première extrémité mâle avec une conicité identique à celle de ladite surface interne d'emmanchement du raccord femelle.

A titre encore plus préférentiel, le raccord mâle est de type « Luer » tel que défini par la norme ISO 59461 de 1986 ou bien de type « Luer Lock » tel que défini par la norme ISO 594-2 de 1998.

Un dispositif de raccordement fluidique selon l'invention, qui est apte à transférer un fluide, tel que des microéchantillons à prélever ou à injecter, et qui est destiné à être raccordé à un premier conduit par une première ouverture de ce dispositif, lequel comporte une seconde ouverture traversée par un second conduit destiné à communiquer avec le premier conduit pour transférer ce fluide, comporte :
- un raccord fluidique femelle qui définit la première ouverture et qui comprend une surface radialement interne d'emmanchement se terminant par une extrémité radiale femelle où débouche le premier conduit, et
- un raccord fluidique mâle qui définit la seconde ouverture en étant emmanché dans le raccord femelle via une surface radialement externe d'emmanchement et qui se termine par une extrémité radiale mâle à l'intérieur du raccord femelle.

Selon l'invention, ce raccord mâle est tel que défini ci-dessus.

De préférence, les raccords mâle et femelle sont tous deux de type « Luer » tels que définis par la norme ISO 59461 de 1986, ou bien tous deux de type « Luer Lock » tels que définis par la norme ISO 594-2 de 1998.

A titre encore plus préférentiel, les raccords mâle et femelle sont tous deux de type « Luer Lock » tels que définis par la norme ISO 594-2 de 1998, le raccord mâle pouvant alors comporter une patte circonférentielle recourbée radialement vers l'intérieur et entourant ladite surface externe d'emmanchement, un rebord d'extrémité en saillie radialement vers l'extérieur que présente le raccord femelle venant se verrouiller entre cette patte et cette surface externe d'emmanchement, pour empêcher les raccords mâle et femelle de s'éloigner l'un de l'autre.

Selon une autre caractéristique de l'invention, avant emmanchement, ledit tube dépasse avantageusement de ladite première extrémité mâle d'une distance axiale initiale qui est supérieure à la profondeur axiale maximale des raccords femelles conformes à l'une ou à l'autre desdites normes, mesurée à partir de ladite extrémité radiale femelle. Comme indiqué précédemment, ce tube est apte à coulisser à l'intérieur du raccord mâle lors de l'emmanchement avec un débattement axial à l'issue du coulissement qui est égal ou supérieur à 8 mm, de sorte que ce tube soit rappelé avec une pression suffisante contre ladite extrémité femelle quelle que soit la profondeur axiale du raccord femelle choisi.

On notera que contrairement aux dispositifs de raccordement standard de type « Luer » ou « Luer Lock » où l'étanchéité se fait par l'ajustement des surfaces d'emmanchement coniques respectives des raccords mâle et femelle, le dispositif de l'invention est conçu pour réaliser l'étanchéité au plus près des entrées/ sorties et directement par ladite extrémité libre du tube.

Avantageusement, ledit premier conduit traversant le raccord femelle est un micro-tube souple adapté pour le prélèvement ou l'injection dudit liquide dans un animal, tel qu'un cathéter souple à implanter dans la veine caudale d'un petit mammifère en vue du prélèvement de microéchantillons sanguins.

Les caractéristiques précitées de la présente invention, ainsi que d'autres, seront mieux comprises à la lecture de la description suivante de plusieurs exemples de réalisation de l'invention, donnés à titre illustratif et non limitatif, ladite description étant réalisée en relation avec le dessin joint, dans lequel :
la figure 1 est une vue partielle en coupe radiale d'un dispositif de raccordement connu à raccords mâle et femelle de type « Luer » qui est destiné à être connecté, d'une part, à un cathéter et, d'autre part, à un micro-tube souple pour le prélèvement de microéchantillons, et
la figure 2 est une vue schématique en section radiale d'un dispositif de raccordement à raccords mâle et femelle selon l'invention de type « Luer Lock » qui sont connectés de manière étanche l'un à l'autre en étant traversés par des microtubes pour le transfert d'un fluide.

Comme illustré à la figure 2, le dispositif de raccordement 101 selon l'invention est dans cet exemple de type « Luer Lock » (en référence à la norme ISO 594-2 de 1998), et il comporte essentiellement :
- un raccord fluidique femelle 102 dans lequel est enfoncé un premier conduit C1, tel qu'un cathéter souple, et qui comprend une surface radialement interne d'emmanchement conique 103 convergeant vers une extrémité radiale femelle 104 où débouche le premier conduit C1 (cette extrémité 104 pouvant être plate ou légèrement conique), et
- un raccord fluidique mâle 105 qui est emmanché dans le raccord femelle 102 via sa surface radialement externe d'emmanchement conique 106 avec la même conicité que la surface interne 103, et qui converge vers une extrémité radiale mâle 107, un second conduit souple C2 tel qu'un micro-tube étant enfoncé dans le raccord mâle 105 en traversant les deux extrémités 107 et 108 de ce dernier et en venant abouter de manière étanche l'extrémité femelle 104.

Selon une caractéristique importante de l'invention, l'extrémité libre 110 du conduit souple C2 est apte à être plaquée de manière étanche contre l'extrémité femelle 104 par un ressort hélicoïdal métallique à compression 111 monté radialement entre le conduit C2 et le raccord mâle 105 et en butée axiale contre une paroi radiale interne d'extrémité 112 du raccord mâle 105 opposée à l'extrémité mâle 107 et contre une portée radiale 113 solidaire en translation du conduit C2, via des moyens de guidage et de maintien 114 dont est pourvu le conduit C2 pour le guider en coulissement axialement à travers le raccord mâle 105 et pour maintenir son extrémité libre 110 en aboutement étanche contre l'extrémité femelle 104.

Dans le mode de réalisation illustré à la figure 2, ces moyens de guidage et de maintien 114 sont constitués d'un manchon tubulaire rigide (tel qu'un manchon métallique ou en matière plastique comme par exemple une résine polymérique), qui est monté solidaire du conduit souple C2 sur la plus grande partie de sa longueur de sorte à coulisser axialement avec lui à travers le raccord mâle 105 en le rigidifiant sur toute la portion où il est enserré par ce manchon 114 (cette portion rigidifiée traverse axialement le raccord mâle 105 en dépassant de ses extrémités 107 et 108 mais en se terminant à distance de l'extrémité libre 110 du conduit C2), et qui présente la portée 113 en forme de collerette circonférentielle pour le ressort 111. Cette collerette 113 peut être obtenue par dudgeonnage dans le cas d'un manchon 114 métallique, ou bien par moulage ou encore par extrusion dans le cas d'un manchon 114 plastique.

Il résulte de ce retrait axial entre le manchon 114 et l'extrémité libre 110 du conduit C2 que ce dernier présente une portion souple d'extrémité apte à assurer l'étanchéité du raccordement sous pression entre le conduit C2 et l'extrémité femelle 104, en supportant la force de rappel F générée par le ressort 111. Cette portion souple d'extrémité du conduit C2 peut par exemple présenter une longueur axiale de 1 mm environ, de manière à assurer cette étanchéité par déformation élastique.

Comme cela sera expliqué ci-après, ce montage coulissant du manchon 114 enserrant le conduit C2 est conçu pour que l'extrémité libre 110 de ce dernier se rapproche de l'extrémité mâle 107 tout en étant rappelée avec une pression suffisante contre l'extrémité femelle 104 par la compression axiale du ressort 111, supprimant ainsi tout volume mort entre le premier conduit C1 et le raccord mâle 105.

Comme illustré à la figure 2, le diamètre extérieur du manchon 114 est choisi inférieur au plus petit diamètre intérieur du raccord femelle 102, que celui-ci soit de type « Luer » ou « Luer Lock ». Ce manchon 114 destiné à éviter le flambage du conduit C2 est formé d'un matériau de rigidité au moins aussi élevée que celle du matériau de ce conduit C2. De préférence, il est formé d'un tube mince métallique ou d'un matériau d'une rigidité suffisante, tel qu'une résine polymérique ou une autre matière plastique. En effet, la conicité des dispositifs « Luer » ou « Luer Lock » impose un diamètre de manchon 114 réduit, particulièrement à proximité du joint étanche entre le conduit C2 et l'extrémité femelle 104 où le diamètre du cône du raccord 102 est le plus faible. Il en résulte le choix d'un manchon 114 de faible épaisseur et donc de rigidité élevée. En position de connexion étanche, il importe que le manchon 114 ne soit pas le siège d'une flexion ou d'un flambage sous la pression de maintien de l'extrémité libre 110 du conduit souple C2 contre l'extrémité femelle 104. On notera que la coupe de cette extrémité libre 110 doit nécessairement présenter une symétrie de révolution, définie préférentiellement par une surface plate perpendiculaire à l'axe de symétrie du conduit C2. Toutefois, selon d'autres modes de réalisation, la coupe de cette extrémité libre 110 pourrait présenter une surface tronconique ou, en variante, un ou plusieurs chanfrein(s), pourvu qu'il(s) satisfasse(nt) à cette condition de symétrie de révolution.

On a obtenu le raccord mâle 105 de la figure 2 à partir d'un raccord mâle « Luer Lock » standard, un alésage axial médian ayant été pratiqué dans ce raccord 105 sur toute sa longueur de manière à y introduire l'ensemble mobile du conduit souple C2 et du manchon de rigidification 114.

Selon une variante non illustrée de la figure 2, ces moyens de guidage et de maintien du conduit C2 pourraient être obtenus par un traitement spécifique durcissant localement ce conduit C2 au voisinage de son extrémité libre 110, tout en laissant à celle-ci une souplesse suffisante pour assurer l'étanchéité en aboutement contre l'extrémité femelle 104.

Plus précisément, le ressort 111 est tel que sa pré-compression assure une étanchéité suffisante via cet aboutement, même pour les raccords femelles 102 utilisés les plus profonds dans la direction axiale, (i.e. pour la course la plus réduite du ressort 111). Quant à la longueur initiale avant emmanchement de dépassement du conduit C2 par rapport à l'extrémité mâle 107, elle est choisie suffisante pour assurer cette étanchéité avec l'extrémité femelle 104, la pression d'aboutement au niveau du joint étanche dépendant de la poussée du ressort 111.

On notera que le dispositif 101 de type « Luer Lock » illustré à la figure 2 est tel que le raccord mâle 105 comporte une patte circonférentielle 115 recourbée radialement vers l'intérieur et entourant la surface externe d'emmanchement 106 du raccord 105, et que le raccord femelle 102 comporte un rebord d'extrémité 116 en saillie radialement vers l'extérieur qui vient se verrouiller entre cette patte 115 et cette surface externe d'emmanchement 106. Ce verrouillage empêche les deux raccords 102 et 105 de s'éloigner l'un de l'autre.

On notera néanmoins qu'un dispositif de raccordement selon l'invention pourrait également concerner des raccords de type « Luer » sans organe de verrouillage, mais qu'il existe dans ce cas un risque de déconnexion intempestive si les raccords n'ont pas été couplés assez fortement entre eux, i.e. si la force de rappel résultant de la pression du ressort 111 et de la pression du fluide devient supérieure à la force d'emmanchement des raccords coniques mâle et femelle.

Un dispositif de raccordement 101 selon l'invention, tel que celui illustré à la figure 2, est amené en position de connexion étanche de la manière suivante.

Avant emmanchement du raccord mâle 105, le conduit C2 pourvu de ses moyens de guidage et de maintien (tels que le manchon 114 dans l'exemple de la figure 2) dépasse de l'extrémité mâle 107 d'une longueur qui est prévue supérieure à la profondeur interne maximale de l'ensemble des raccords femelles 102 utilisables.

Lorsque le raccord mâle 105 est enfoncé par emmanchement dans le raccord femelle 102 choisi, l'extrémité libre 110 souple du conduit C2 est maintenue en contact étanche avec l'extrémité femelle 104 via le ressort 111 qui assure une poussée suffisante (flèche F) pour y plaquer de manière étanche cette extrémité 110.

A cet effet, lors de l'assemblage du dispositif 101, le ressort 111 reçoit une précontrainte de manière à assurer cette poussée suffisante même sur les raccords femelles 102 les plus profonds, cette poussée étant calculée de manière à garantir une tenue en pression du joint minimale de 330 kPa selon la norme « Luer Lock » ISO 594-2 de 1998, et cela quel que soit le raccord femelle 102 choisi. Ainsi, pour un raccord femelle 102 relativement profond, cette pression sera par exemple de 400 kPa, alors que le même raccord mâle 105 monté sur un raccord femelle 102 « Luer Lock » moins profond aura son ressort 111 davantage comprimé, augmentant en proportion la pression maximale admissible par le joint à l'aboutement, par exemple de 600 kPa (pour calculer la pression de tenue du joint, on divise la force de rappel exercée par le ressort 111, qui est fonction de sa compression, par la surface du joint définie par la surface de la coupe du conduit souple C2 en son extrémité libre 110).

On comprendra qu'en utilisant un ressort 111 de grande longueur, on diminue la différence de pression sur ce joint pour une connexion sur un raccord femelle « Luer » ou « Luer Lock » peu profond, en comparaison d'un raccord femelle du même type mais plus profond.

Quant au débattement du manchon 114 suite à son coulissement à l'intérieur du raccord mâle 105, il est prévu supérieur à la différence maximale de profondeur interne - de 6 mm environ - entre les différents types de raccords femelles de la gamme « Luer » ou « Luer Lock », et ce débattement est en conséquence prévu supérieur à 8 mm.

L'application préférentielle d'un dispositif de raccordement selon l'invention concerne une ligne de cathétérisation pour microéchantillons de fluide liquide. Grâce à ce dispositif, toute ligne de cathétérisation utilisant des raccords femelles « Luer » ou « Luer Lock » standards permet le transfert de ces microéchantillons sans risque de mélange de ceux-ci, ce qui permet une traçabilité parfaite (les microéchantillons en sortie de ligne étant conformes à ceux en entrée de ligne). En outre, on n'a pas besoin d'attendre le prélèvement de trop nombreux microéchantillons pour commencer les mesures ou analyses, et cela évite de gaspiller un volume de liquide correspondant au volume mort de l'art antérieur. A titre d'exemple, pour le raccordement à un cathéter C1 de 1 mm de diamètre intérieur, on prendra de préférence un tube souple C2 de diamètre interne égal à 1 mm.

On notera cependant qu'un dispositif de raccordement selon l'invention pourrait également être utilisé dans certaines applications mettant en jeu des échantillons de volumes élevés ou ne nécessitant pas de traçabilité temporelle et spatiale, avec comme avantage résultant de cette autre utilisation la minimisation des parties souillées et de la quantité résiduelle de fluide après manipulation, dans le cas du traitement de liquides particulièrement surveillés (e.g. du sang contaminé, des liquides radioactifs, etc.).

## Revendications

1. Raccord fluidique mâle (105) destiné à être emmanché par enfoncement dans un raccord fluidique femelle (102) raccordé à un premier conduit (C1) avec lequel est destiné à communiquer un second conduit (C2) formé d'un tube souple qui est enfoncé dans le raccord mâle axialement et traversant ledit raccord mâle, pour former un dispositif de raccordement fluidique (101) apte à transférer un fluide, tel que des microéchantillons à prélever ou à injecter, le raccord fluidique femelle (102) comprenant une surface radialement interne d'emmanchement conique (103) se terminant par une extrémité radiale femelle (104) et convergeant vers cette dernière, le raccord mâle se terminant par une première extrémité radiale mâle (107) destinée à se trouver à l'intérieur du raccord femelle, le premier conduit (C1) débouche à l'extrémité radiale femelle (104) et en ce que le raccord mâle (105) comprend une surface externe d'emmanchement (106) par enfoncement **caractérisé en ce que**, le second conduit (C2) est enfoncé au-delà de ladite première extrémité mâle et qui se termine par une extrémité libre (110), coupée selon une symétrie cylindrique, et apte à être plaquée de manière étanche contre ladite extrémité femelle (104) par des moyens de rappel (111) intégrés au raccord mâle, les moyens de rappel (111) comprennent un ressort hélicoïdal à compression monté radialement entre ledit tube souple (C2) et le raccord mâle (105), le ressort hélicoïdal à compression étant montés en butée axiale d'une part, contre une paroi radiale d'extrémité (112) du raccord mâle (105) opposée à ladite première extrémité mâle (107) et d'autre part, contre au moins une portée radiale (113) qui est solidaire en translation dudit tube (C2), ces moyens de rappel coopérant lors de l'emmanchement par enfoncement avec des moyens de guidage et de maintien (114) prévus dans le raccord mâle pour guider ce tube en coulissement axial à travers le raccord mâle en rapprochant ladite extrémité libre de ladite première extrémité mâle tout en maintenant cette extrémité libre contre ladite extrémité femelle, de sorte à supprimer tout volume mort entre le premier conduit et le raccord mâle et pour maintenir l'extrémité libre (110) en aboutement étanche contre l'extrémité femelle (104) et **en ce que** le raccord fluidique mâle (105) étant emmanché dans le raccord femelle (102) via sa surface externe d'emmanchement (106), la surface externe d'emmanchement (106) étant conique de même conicité que la surface interne d'emmanchement (103) du raccord femelle (102), et qui converge vers l'extrémité radiale mâle (107), le second conduit (C2) étant enfoncé dans le raccord mâle (105) en traversant les deux extrémités (107, 108) du raccord mâle (105), et en venant abouter de manière étanche l'extrémité femelle (104).

2. Raccord fluidique mâle (105) selon la revendication 1, **caractérisé en ce que** lesdits moyens de rappel (111) sont aptes à être comprimés axialement lors du coulissement dudit tube (C2) vers l'intérieur du raccord mâle, de telle sorte que ladite extrémité libre (110) soit plaquée contre ladite extrémité femelle (104) avec une force résultant au moins en partie de la compression des moyens de rappel (111) due au débattement dudit tube lors de son coulissement, ce débattement étant prévu supérieur à la profondeur axiale du raccord femelle (102) mesurée à partir de ladite extrémité femelle.

3. Raccord fluidique mâle (105) selon la revendication 2, **caractérisé en ce que** ladite force de plaquage résulte non seulement de la compression desdits moyens de rappel (111) lors du débattement dudit tube (C2) mais en outre d'une précontrainte appliquée à ces moyens de rappel avant montage du raccord mâle dans le raccord femelle, cette précontrainte étant telle que, dès le début de son coulissement, ladite extrémité libre (110) dudit tube est sensiblement apte à assurer l'étanchéité vis-à-vis du raccord femelle (102), une compression réduite desdits moyens de rappel lors dudit coulissement permettant de conférer à cette force de plaquage une intensité suffisante pour assurer cette étanchéité.

4. Raccord fluidique mâle (105) selon une des revendications précédentes, **caractérisé en ce que** lesdits moyens de guidage et de maintien (114) sont conçus pour rigidifier ledit tube (C2) sur une portion rigidifiée de ce dernier s'étendant axialement à l'intérieur du raccord mâle et se terminant au-delà de ladite première extrémité mâle (107) à distance de ladite extrémité libre (110), de sorte que ce tube présente une portion souple d'extrémité de rigidité inférieure à celle de cette portion rigidifiée et apte à assurer l'étanchéité du raccordement entre ladite extrémité libre et ladite extrémité femelle (104) en supportant la force de rappel (F) générée par lesdits moyens de rappel (111) contre ladite extrémité femelle, ces portions respectivement rigidifiée et souple étant adaptées pour éviter le flambage dudit tube à l'intérieur du raccord mâle et contre cette extrémité libre.

5. Raccord fluidique mâle (105) selon la revendication 4, **caractérisé en ce que** lesdits moyens de guidage et de maintien (114) comprennent un manchon tubulaire rigide (114) monté solidaire dudit tube (C2) en l'enserrant sur ladite portion rigidifiée, tel qu'un manchon métallique ou en matière plastique, ladite portion souple d'extrémité dudit tube étant dépourvue de ce manchon, lequel est monté coulissant axialement à l'intérieur du raccord mâle en traversant ladite première extrémité mâle (107) et une seconde extrémité mâle opposée (108) du raccord mâle.

6. Raccord fluidique mâle (105) selon les revendications 1 et 5, **caractérisé en ce que** ladite portée radiale (113) pour lesdits moyens de rappel (111) est formée par une collerette circonférentielle que présente ledit manchon (114).

7. Raccord fluidique mâle (105) selon la revendication 4, **caractérisé en ce que** ladite portion rigidifiée fait partie intégrante dudit tube (C2) en présentant une rigidité supérieure à celle de ladite portion souple d'extrémité de ce tube, cette portion rigidifiée étant obtenue par un traitement de durcissement local de ce tube.

8. Raccord fluidique mâle (105) selon une des revendications précédentes, **caractérisé en ce que** lesdits moyens de guidage et de maintien (114) autorisent en outre une rotation libre dudit tube (C2) à l'intérieur du raccord mâle.

9. Raccord fluidique mâle (105) selon une des revendications précédentes, **caractérisé en ce que** ladite surface externe d'emmanchement (106) du raccord mâle (105) est une surface conique qui converge vers ladite première extrémité mâle (107) avec une conicité identique à celle de ladite surface interne d'emmanchement (103) du raccord femelle (102), et **en ce que** de préférence ce raccord mâle est de type « Luer » tel que défini par la norme ISO 59461 de 1986 ou bien de type « Luer Lock » tel que défini par la norme ISO 594-2 de 1998.

10. Dispositif de raccordement fluidique (101) qui est apte à transférer un fluide, tel que des microéchantillons à prélever ou à injecter, et qui est destiné à être raccordé à un premier conduit (C1) par une première ouverture de ce dispositif, lequel comporte une seconde ouverture traversée par un second conduit (C2) destiné à communiquer avec le premier conduit pour transférer ce fluide, le dispositif comportant :
- un raccord fluidique femelle (102) qui définit ladite première ouverture et qui comprend une surface interne d'emmanchement par enfoncement (103) se terminant par une extrémité radiale femelle (104) où débouche ce premier conduit, et
- un raccord fluidique mâle (105) qui définit ladite seconde ouverture en étant emmanché par enfoncement dans le raccord femelle via une surface externe d'emmanchement par enfoncement (106) et qui se termine par une première extrémité radiale mâle (107) à l'intérieur du raccord femelle, **caractérisé en ce que** ce raccord mâle est tel que défini à l'une des revendications précédentes.

11. Dispositif de raccordement fluidique (101) selon la revendication 10, **caractérisé en ce que** les raccords mâle (105) et femelle (102) sont tous deux de type « Luer » tels que définis par la norme ISO 59461 de 1986, ou bien tous deux de type « Luer Lock » tels que définis par la norme ISO 594-2 de 1998, avec de préférence dans ce second cas le raccord mâle qui comporte une patte circonférentielle (115) recourbée radialement vers l'intérieur et entourant ladite surface externe d'emmanchement (106), un rebord d'extrémité (116) en saillie radialement vers l'extérieur que présente le raccord femelle venant se verrouiller entre cette patte et cette surface externe d'emmanchement.

12. Dispositif de raccordement fluidique (101) selon la revendication 11, **caractérisé en ce que**, avant emmanchement, ledit tube (C2) dépasse de ladite première extrémité mâle (107) d'une distance axiale initiale qui est supérieure à la profondeur axiale maximale des raccords femelles (102) conformes à l'une ou à l'autre desdites normes.

13. Dispositif de raccordement fluidique (101) selon la revendication 12, **caractérisé en ce que** ledit tube (C2) est apte à coulisser à l'intérieur du raccord mâle (105) lors de l'emmanchement avec un débattement axial à l'issue du coulissement qui est égal ou supérieur à 8 mm, de sorte que ce tube soit rappelé avec une pression suffisante contre ladite extrémité femelle (104) quelle que soit la profondeur axiale du raccord femelle (102) choisi.

14. Dispositif de raccordement fluidique (101) selon une des revendications 10 à 13, **caractérisé en ce que** ledit premier conduit (C1) est un micro-tube souple adapté pour le prélèvement ou l'injection dudit liquide dans un animal, tel qu'un cathéter souple à implanter dans la veine caudale d'un petit mammifère en vue du prélèvement de microéchantillons sanguins.

## Patentansprüche

1. Fluidischer Steckanschluss (105), der dazu bestimmt ist, durch Eindrücken in einen fluidischen Buchsenanschluss (102), der an eine erste Leitung (C1) angeschlossen ist, eingepasst zu werden, mit der eine zweite Leitung (C2) zu kommunizieren bestimmt ist, aus einem flexiblen Rohr gebildet, das axial in den fluidischen Steckanschluss eingedrückt ist, und durch den Steckanschluss führt, um eine fluidische Kopplungsvorrichtung (101) zu bilden, die imstande ist, ein Fluid, wie zu entnehmende oder zu injizierende Mikrostichproben zu transferieren, wobei der fluidische Buchsenanschluss (102) eine radial innere Oberfläche zum konischen Einpassen (103) umfasst, die in einem radialen Buchsenende (104) abschließt und zu dieser Letzteren zusammenläuft, wobei der Steckanschluss in einem ersten radialen Steckende (107) abschließt, das dazu bestimmt ist, sich im Inneren des Buchsenanschlusses zu befinden, wobei die erste Leitung (C1) in das radiale Buchsenende (104) mündet, und dadurch, dass der Steckanschluss (105) eine äußere Oberfläche zum Einpassen (106) durch Eindrücken umfasst
**dadurch gekennzeichnet, dass** die zweite Leitung (C2) über das erste Steckende hinaus eingedrückt ist, und sie durch ein freies Ende (110) abschließt, das entsprechend einer zylindrischen Symmetrie abgeschnitten ist und imstande ist, durch im Steckanschluss integrierte Rückholmittel (111) dicht an dem Buchsenende (104) angelegt zu werden, wobei die Rückholmittel (111) eine Spiraldruckfeder umfassen, die radial zwischen dem flexiblen Rohr (C2) und dem Steckanschluss (105) montiert ist, wobei die Spiraldruckfeder einerseits auf axialen Anschlag an einer radialen Endwand (112) des Steckanschlusses (105) gegenüber dem ersten Steckende (107), und andererseits an mindestens einer radialen Auflagefläche (113) montiert ist, die translationsfest mit dem Rohr (C2) ist, wobei diese Rückholmittel beim Einpassen durch Eindrücken mit Führungs- und Haltemitteln (114) zusammenwirken, die in dem Steckanschluss vorgesehen sind, um dieses Rohr durch axiales Gleiten durch den Steckanschluss hindurchführen, und dabei dieses freie Ende an das erste Steckende annähern und dabei dieses freie Ende am Buchsenende halten, um das gesamte Totvolumen zwischen der ersten Leitung und dem Steckanschluss zu entfernen und um das freie Ende (110) in dichter Zusammenfügung am Buchsenende (104) zu halten, und dadurch, dass der fluidische Steckanschluss (105) über seine äußere Oberfläche zum Einpassen (106) in den Buchsenanschluss (102) eingepasst wird, wobei die äußere Oberfläche zum Einpassen (106) mit derselben Konizität konisch ist, wie die innere Oberfläche zum Einpassen (103) des Buchsenanschlusses (102) und die zum radialen Steckende (107) zusammenläuft, wobei die zweite Leitung (C2) in den Steckanschluss (105) eingedrückt wird, indem sie durch die beiden Enden (107, 108) des Steckanschlusses (105) führt und das Buchsenende (104) dicht zusammenfügt.

2. Fluidischer Steckanschluss (105) nach Anspruch 1, **dadurch gekennzeichnet, dass** die Rückholmittel (111) imstande sind, beim Gleiten des Rohres (C2) ins Innere des Steckanschlusses axial derart komprimiert zu werden, sodass das freie Ende (110) mit einer Kraft an das Buchsenende (104) gepresst wird, die mindestens teilweise aus der Kompression der Rückholmittel (111) aufgrund des Federwegs des Rohres bei seinem Gleiten resultiert, wobei dieser Federweg größer vorgesehen ist als die axiale Tiefe des Buchsenanschlusses (102), die aus dem Buchsenende heraus gemessen wird.

3. Fluidischer Steckanschluss (105) nach Anspruch 2, **dadurch gekennzeichnet, dass** die Presskraft nicht nur aus der Kompression der Rückholmittel (111) beim Federweg des Rohres (C2) resultiert, sondern weiter aus einer Vorspannung, die auf diese Rückholmittel vor der Montage des Steckanschlusses in den Buchsenanschluss angelegt wird, wobei diese Vorspannung derart ist, dass das freie Ende (110) des Rohres bereits zu Beginn seines Gleitens im Wesentlichen imstande ist, für die Dichtheit gegenüber dem Buchsenanschluss (102) zu sorgen, wobei es eine reduzierte Kompression der Rückholmittel bei dem Gleiten ermöglicht, dieser Presskraft eine ausreichende Intensität zur Gewährleistung dieser Dichtheit zu verleihen.

4. Fluidischer Steckanschluss (105) nach einem der vorstehenden Ansprüche, **dadurch gekennzeichnet, dass** die Führungs- und Haltemittel (114) gestaltet sind, um das Rohr (C2) an einem versteiften Abschnitt dieses Letzteren, der sich axial im Inneren des Steckanschlusses erstreckt, und über das erste Steckende (107) hinaus auf Abstand von dem freien Ende (110) abschließt, zu versteifen, sodass dieses Rohr einen flexiblen Endabschnitt mit einer Steifigkeit aufweist, die geringer als jene dieses versteiften Abschnitts ist und imstande ist, für die Dichtheit des Anschlusses zwischen dem freien Ende und dem Buchsenende (104) durch Stützen der Rückholkraft (F) zu sorgen, die durch die Rückholmittel (111) gegen das Buchsenende erzeugt wird, wobei diese jeweils versteiften und flexiblen Abschnitte ausgeführt sind, um das Knicken des Rohres im Inneren des Steckanschlusses und gegen dieses freie Ende zu vermeiden.

5. Fluidischer Steckanschluss (105) nach Anspruch 4, **dadurch gekennzeichnet, dass** die Führungs- und Haltemittel (114) eine steife Rohrmuffe (114) umfassen, die kraftschlüssig mit dem Rohr (C2) montiert ist, indem sie es an dem versteiften Abschnitt wie eine Muffe aus Metall oder aus Kunststoffmaterial einspannt, wobei der flexible Endabschnitt des Rohres nicht mit dieser Muffe versehen ist, welche axial gleitend im Inneren des Steckanschlusses montiert ist, indem sie durch das erste Steckende (107) und ein zweites gegenüberliegendes Steckende (108) des Steckanschlusses führt.

6. Fluidischer Steckanschluss (105) nach den Ansprüchen 1 und 5, **dadurch gekennzeichnet, dass** die radiale Auflagefläche (113) für die Rückholmittel (111) durch einen umlaufenden Kragen gebildet wird, den die Muffe (114) aufweist.

7. Fluidischer Steckanschluss (105) nach Anspruch 4, **dadurch gekennzeichnet, dass** der versteifte Abschnitt integraler Bestandteil des Rohres (C2) ist, indem er eine Steifigkeit größer als jene des flexiblen Endabschnitts dieses Rohres aufweist, wobei dieser versteifte Abschnitt durch eine lokale Härtebehandlung dieses Rohres erhalten wird.

8. Fluidischer Steckanschluss (105) nach einem der vorstehenden Ansprüche, **dadurch gekennzeichnet, dass** die Halte- und Führungsmittel (114) weiter eine freie Drehung des Rohres (C2) im Inneren des Steckanschlusses erlauben.

9. Fluidischer Steckanschluss (105) nach einem der vorstehenden Ansprüche, **dadurch gekennzeichnet, dass** die äußere Oberfläche zum Einpassen (106) des Steckanschlusses (105) eine konische Oberfläche ist, die mit einer identischen Konizität zum ersten Steckende (107) zusammenläuft, wie jene der inneren Oberfläche zum Einpassen (103) des Buchsenanschlusses (102), und dadurch, dass dieser Steckanschluss vorzugsweise vom "Luer"-Typ ist, wie durch die Norm ISO 59461 aus 1986 definiert, oder vom "Luer Lock"-Typ, wie durch die Norm ISO 594-2 aus 1998 definiert.

10. Fluidische Kopplungsvorrichtung (101), die imstande ist, ein Fluid, wie zu entnehmende oder zu injizierende Mikrostichproben zu transferieren, und die dazu bestimmt ist, an eine erste Leitung (C1) durch eine erste Öffnung dieser Vorrichtung angeschlossen zu werden, welche eine zweite Öffnung beinhaltet, durch die eine zweite Leitung (C2) führt, die dazu bestimmt ist, mit der ersten Leitung zu kommunizieren, um dieses Fluid zu transferieren, wobei die Vorrichtung beinhaltet:
- einen fluidischen Buchsenanschluss (102), der die erste Öffnung definiert und der eine innere Oberfläche zum Einpassen durch Einpressen (103) umfasst, die durch ein radiales Buchsenende (104) abschließt, wo diese erste Leitung einmündet, und
- einen fluidischen Steckanschluss (105), der die zweite Öffnung definiert, indem er durch Einpressen in den Buchsenanschluss über eine äußere Oberfläche zum Einpassen (106) durch Eindrücken eingepasst wird, und durch ein erstes radiales Steckende (107) im Inneren des Buchsenendes abschließt,
**dadurch gekennzeichnet, dass** der Steckanschluss nach einem der vorstehenden Ansprüche definiert ist.

11. Fluidische Kopplungsvorrichtung (101) nach Anspruch 10, **dadurch gekennzeichnet, dass** der Steck- (105) und Buchsenanschluss (102) beide vom "Luer"-Typ, wie durch die Norm ISO 59461 aus 1986 definiert, sind, oder aber beide vom "Luer Lock"-Typ, wie durch die Norm ISO 594-2 aus 1998 definiert, sind, mit vorzugsweise in diesem zweiten Fall dem Steckanschluss, der eine umlaufende Lasche (115) beinhaltet, die radial nach innen gekrümmt ist und die äußere Oberfläche zum Einpassen (106) umgibt, wobei eine Endkante (116), radial nach außen überstehend, welche der Buchsenanschluss aufweist, zwischen dieser Lasche und dieser äußeren Oberfläche zum Einpassen abriegelt.

12. Fluidische Kopplungsvorrichtung (101) nach Anspruch 11, **dadurch gekennzeichnet, dass** das Rohr (C2) vor dem Einpassen das erste Steckende (107) um einen anfänglichen axialen Abstand überschreitet, der entsprechend der einen oder anderen Norm größer als die maximale axiale Tiefe der Buchsenanschlüsse (102) ist.

13. Fluidische Kopplungsvorrichtung (101) nach Anspruch 12, **dadurch gekennzeichnet, dass** das Rohr (C2) imstande ist, beim Einpassen mit einem axialen Federweg am Ende des Gleitens ins Innere des Steckanschlusses (105) zu gleiten, der größer oder gleich 8 mm ist, sodass dieses Rohr mit einem ausreichenden Druck an das Buchsenende (104) unabhängig von der axialen Tiefe des gewählten Buchsenanschlusses (102) zurückgeholt wird.

14. Fluidische Kopplungsvorrichtung (101) nach einem der Ansprüche 10 bis 13, **dadurch gekennzeichnet, dass** die erste Leitung (C1) ein flexibles Mikrorohr ist, das zum Entnehmen oder Injizieren der Flüssigkeit in einem Tier ausgeführt ist, wie ein flexibler Katheter, der in die Schwanzvene eines kleinen Säugetieres im Hinblick auf die Entnahme von Mikroblutproben einzusetzen ist.

## Claims

1. Male fluidic coupling (105) intended to be press-fitted into a female fluidic coupling (102) coupled to a first pipe (C1) intended to communicate with a second pipe (C2) formed from a flexible tube that is pushed into the male coupling axially and passing through said male coupling, to form a fluidic coupling device (101) capable of transferring a fluid, such as microsamples to be taken or injected, the female fluidic coupling (102) comprising a radially internal tapered press-fitting surface (103) ending in a female radial end (104) and converging towards the latter, the male coupling ending in a first radial male end (107) intended to lie inside the female coupling, the first pipe (C1) leading to the female radial end (104) and in that the male coupling (105) comprises an external press-fitting surface (106) by pressing,
**characterised in that**, the second pipe (C2) is pushed beyond said first male end and which ends in a free end (110), cut according to a cylindrical symmetry, and capable of being pressed in a sealed manner against said female end (104) by return means (111) integrated into the male coupling, the return means (111) comprise a helical compression spring mounted radially between said flexible tube (C2) and the male coupling (105), with the helical compression spring being mounted in axial abutment on the one hand, against a radial end wall (112) of the male coupling (105) opposite said first male end (107) and on the other hand, against at least one radial surface (113) which is secured in translation with said tube (C2), these return means cooperating during the press-fitting by pushing with guiding and retaining means (114) provided in the male coupling to guide this tube in sliding axially through the male coupling by bringing together said free end with said first male end while still maintaining this free end against said female end, so as to suppress any dead volume between the first pipe and the male coupling and to maintain the free end (110) in a sealed abutment against the female end (104) and **in that** the male fluidic coupling (105) being press-fitted in the female coupling (102) via the external press-fitting surface (106) thereof, the external press-fitting surface (106) being tapered with the same taper as the internal press-fitting surface (103) of the female coupling (102), and which converges towards the male radial wall (107), the second pipe (C2) being pushed into the male coupling (105) by passing through the two ends (107, 108) of the male coupling (105), and abutting in a sealed manner with the female end (104).

2. Male fluidic coupling (105) according to claim 1, **characterised in that** said return means (111) are capable of being compressed axially during the sliding of said tube (C2) towards the inside of the male coupling, such that said free end (110) is pressed against said female end (104) with a force resulting at least partially from the compression of the return means (111) due to the clearance of said tube during the sliding thereof, this clearance being provided greater than the axial depth of the female coupling (102) measured from said female end.

3. Male fluidic coupling (105) according to claim 2, **characterised in that** said pressing force is the result not only of the compression of said return means (111) during the clearance of said tube (C2) but furthermore from a prestress applied to the return means before mounting of the male coupling in the female coupling, this prestress being such that, as soon as it starts to slide, said free end (110) of said tube is substantially capable of ensuring the seal with regards to the female coupling (102), a reduced compression of said return means during said sliding making it possible to confer upon this pressing force an intensity that is sufficient to ensure this seal.

4. Male fluidic coupling (105) according to one of the preceding claims, **characterised in that** said guiding and retaining means (114) are designed to rigidify said tube (C2) over a rigidified portion of the latter extending axially inside the male coupling and ending beyond said first male end (107) at a distance from said free end (110), such that this tube has a flexible end portion with a rigidity that is less than that of this rigidified portion and capable of ensuring the sealing of the coupling between said free end and said female end (104) by supporting the return force (F) generated by said return means (111) against said female end, with these respectively rigidified and flexible portions being suitable for preventing the bowing of said tube inside the male coupling and against this free end.

5. Male fluidic coupling (105) according to claim 4, **characterised in that** said guiding and retaining means (114) comprises a rigid tubular sleeve (114) mounted secured with said tube (C2) by surrounding it on said rigidified portion, such as a metal or plastic material sleeve, said flexible end portion of said tube being devoid of this sleeve, which is mounted axially sliding inside the male coupling by passing through said first male end (107) and a second male end opposite (108) the male coupling.

6. Male fluidic coupling (105) according to claims 1 and 5, **characterised in that** said radial surface (113) for said return means (111) is formed by a circumferential collar of said sleeve (114).

7. Male fluidic coupling (105) according to claim 4, **characterised in that** said rigidified portion is an integral part of said tube (C2) by having a rigidity that is greater than that of said flexible end portion of this tube, with this rigidified portion being obtained by a local hardening treatment of this tube.

8. Male fluidic coupling (105) according to one of the preceding claims, **characterised in that** said guiding and retaining means (114) further enables free rotation of said tube (C2) inside the male coupling.

9. Male fluidic coupling (105) according to one of the preceding claims, **characterised in that** said external press-fitting surface (106) of the male coupling (105) is a tapered surface that converges towards said first male end (107) with a taper identical to that of said internal press-fitting surface (103) of the female coupling (102), and **in that** preferably this male coupling is of the "Luer" type such as defined by the standard ISO 59461 of 1986 or of the "Luer Lock" type such as defined by the standard ISO 594-2 of 1998.

10. Fluidic coupling device (101) which is capable of transferring a fluid, such as microsamples to be taken or injected, and which is intended to be coupled to a first pipe (C1) by a first opening of this device, which comprises a second opening passed through by a second pipe (C2) intended to communicate with the first pipe in order to transfer this fluid, the device comprising:
- a female fluidic coupling (102) which defines said first opening and which comprises an internal press-fitting surface by pushing (103) ending in a female radial end (104) where this first pipe emerges, and
- a male fluidic coupling (105) which defines said second opening by being press-fitted by pushing in the female coupling via an external press-fitting surface by pushing (106) and which ends in a first radial male end (107) inside the female coupling,
**characterised in that** this male coupling is such as defined in one of the preceding claims.

11. Fluidic coupling device (101) according to claim 10, **characterised in that** the male (105) and female (102) couplings are both of the "Luer" type such as defined in the standard ISO 59461 of 1986, or both of the "Luer Lock" type such as defined in the standard ISO 594-2 of 1998, with preferably in this second case, the male coupling which comprises a circumferential tab (115) curved radially inwards and surrounding said external press-fitting surface (106), an end edge (116) protruding radially outwards on the female coupling comes to lock between this tab and this external press-fitting surface.

12. Fluidic coupling device (101) according to claim 11, **characterised in that**, before press-fitting, said tube (C2) exceeds said first male end (107) by an initial axial distance that is greater than the maximum axial depth of the female couplings (102) according to one or the other of said standards.

13. Fluidic coupling device (101) according to claim 12, **characterised in that** said tube (C2) is capable of sliding inside the male coupling (105) during the press-fitting with an axial clearance at the end of the sliding which is equal to or greater than 8mm, such that this tube is returned with sufficient pressure against said female end (104) regardless of the axial depth of the female coupling (102) selected.

14. Fluidic coupling device (101) according to one of claims 10 to 13, **characterised in that** said first pipe (C1) is a flexible micro-tube suitable for taking or injecting said liquid in an animal, such as a flexible catheter to be implanted in a tail vein of a small mammal for the purpose of taking microsamples of blood.
